# EUROPÄISCHE PATENTANMELDUNG

(11) **EP 1 223 161 A2**
(43) Veröffentlichungstag der Anmeldung: **17.07.2002**
(21) Anmeldenummer: 01129789.2
(22) Anmeldetag: 14.12.2001
(51) Int. Cl.: C07C 209/90, C07C 41/58, C07C 319/26, C09K 15/00, C07B 63/04

(54) **Verfahren zur Stabilisierung und/oder Senkung der Farbzahl von Alkenylverbindungen**

(30) Priorität: 10.01.2001 DE 10100751
(71) Anmelder: BASF AKTIENGESELLSCHAFT, 67056 Ludwigshafen (DE)
(72) Erfinder: Lorenz, Rudolf Erich, 67069 Ludwigshafen (DE); Böttcher, Arnd, Dr., 67227 Frankenthal (DE); Becker, Heicke, Dr., 67227 Frankenthal (DE); Pinkos, Rolf, Dr., 67089 Bad Dürkheim (DE)

(57) **Zusammenfassung**

Verfahren zur Stabilisierung und/oder Senkung der Farbzahl von Alkenylverbindungen mit einem zwei- oder dreibindigen Heteroatom in α-Stellung zur Doppelbindung, bei dem man die Alkenylverbindungen mit einem Oxidationsmittel versetzt.

## Beschreibung

Die vorliegende Erfindung betrifft ein Verfahren zur Stabilisierung und/oder Senkung der Farbzahl von Alkenylverbindungen mit einem zwei- oder dreibindigen Heteroatom in α-Stellung zur Doppelbindung, insbesondere von Alkenylverbindungen der allgemeinen Formeln (Ia) oder (Ib) 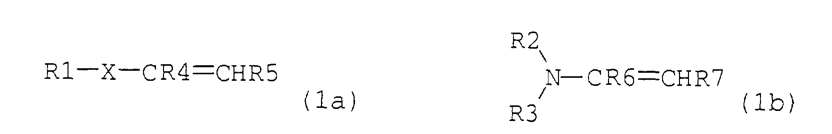 in denen X ein zweibindiges Heteroatom, R¹, R² und R³ jeweils unabhängig voneinander ein Kohlenstoff enthaltender organischer Rest, wobei R² und R³ auch miteinander verbunden sein können, und R⁴, R⁵, R⁶ und R⁷ jeweils unabhängig voneinander Wasserstoff oder ein Kohlenwasserstoffrest, bedeuten.

Alkenylverbindungen finden unter anderem Verwendung als Monomerbausteine in Oligomeren, Polymeren und Copolymeren. Somit finden Alkenylverbindungen beispielsweise Eingang in der Herstellung von Papierbeschichtungen, Klebstoffen, Druckfarben, Waschmitteln, Motorölzusätzen, Textilhilfsmitteln, strahlungshärtenden Lacken, Kosmetika, Pharmazeutika, Hilfsmitteln für die Erdölförderung oder Chemikalien für fotographische Anwendungen.

Alkenylverbindungen werden technisch durch verschiedene Verfahren, wie beispielsweise durch Addition von Alkinen (Alkenylierung), Übertragung von Alkenylgruppen, Eliminierung unter Ausbildung der Doppelbindung oder oxidative Addition von Alkenen gewonnen. Eine Übersicht betreffend die Herstellung von Vinylethern und Vinylestern ist in Ullmann's Encyclopedia of Industrial Chemistry, 6^{th} edition, 1999 Electronic Release, Chapter "VINYL ETHERS" und Chapter "VINYL ESTERS" gegeben.

W. Reppe et al., Justus Liebigs Ann. Chem. Bd. 601, 1956, Seiten 81 bis 138 beschreibt die Herstellung von Vinylethern, Vinylestern, Vinylaminen, Vinyl-N-heterocyclen und Vinylamiden durch Umsetzung von Ethin mit den entsprechenden Alkoholen, Carbonsäuren, Aminen, NH-Heterocyclen und Amiden in Gegenwart basischer Katalysatoren.

Wie aus den zitierten Schriften hervorgeht, schließt sich an den eigentlichen Syntheseschritt üblicherweise eine destillative Reinigung an, bei der die gewünschten Produkte durch Kondensation aus der Gasphase in hoher Reinheit gewonnen werden können. Reinheiten von deutlich über 99% sind somit bei sehr vielen Alkenylverbindungen problemlos erreichbar, was für eine Vielzahl von Anwendungen auch vollkommen ausreicht. Um unerwünschte Reaktionen wie etwa Zersetzung, Oligomerisierung oder Polymerisierung bei der Lagerung und beim Transport zu unterbinden, wird üblicherweise ein Stabilisator zugegeben. Ein häufig eingesetzter Stabilisator ist N,N'-Bis(1-methylpropyl)-1,4-phenylendiamin, welcher von der BASF AG unter dem Handelsnamen Kerobit® BPD vertrieben wird. Weitere bekannte Stabilisatoren sind beispielsweise Alkalihydroxide oder Phenothiazin-Derivate.

Bei Anwendungen, bei denen die Eigenfärbung des Produkts möglichst gering sein sollte, wie beispielsweise im kosmetischen oder fotographischen Bereich oder bei Papierbeschichtungen, ist neben einer hohen chemischen Reinheit auch eine sehr hohe Reinheit in Bezug auf farbgebende Verunreinigungen gefragt. Bei farbgebenden Verunreingungen reichen im Allgemeinen Mengen von wenigen Gew.-ppb aus, um deutliche Verfärbungen des Produkts zu erreichen. Um Alkenylverbindungen mit sehr niedriger Farbzahl zu erhalten, sind üblicherweise aufwendige Reinigungsverfahren wie beispielsweise mehrfache Destillation oder Kristallisation erforderlich.

Sowohl in Gegenwart als auch in Abwesenheit eines Stabilisators zeigen die Alkenylverbindungen in der Regel eine Tendenz zur Verfärbung. Dies führt dazu, daß das Produkt nach der Lagerung beziehungsweise dem Transport eine deutlich dunklere Farbe aufweist als vorher. Dieses nachteilige Verhalten hat somit einen entscheidenden Einfluß auf die Produktqualität, was in der Praxis zur Folge hat, daß entweder eine schlechtere Produktqualität in Kauf genommen werden muß oder vor der Verwendung der Alkenylverbindungen eine erneute aufwendige Reinigung zu erfolgen hat.
Es bestand daher die Aufgabe, ein Verfahren zur Stabilisierung und/oder Senkung der Farbzahl von Alkenylverbindungen zu finden, welches die oben genannten Nachteile nicht mehr besitzt und unter Vermeidung eines hohen Aufwandes zu farbzahlstabilisierten Alkenylverbindungen mit sehr niedriger Farbzahl führt, welche auch nach längerer, mehrmonatiger Lagerung keine oder nur eine sehr geringe Tendenz zur Verfärbung zeigen.

Es wurde nun überraschenderweise ein Verfahren zur Stabilisierung und/oder Senkung der Farbzahl von Alkenylverbindungen mit einem zwei- oder dreibindigen Heteroatom in α-Stellung zur Doppelbindung gefunden, das dadurch gekennzeichnet ist, daß man die Alkenylverbindungen mit einem Oxidationsmittel versetzt.

Unter dem Begriff Oxidationsmittel sind Elemente und Verbindungen zu verstehen, welche bestrebt sind, durch Aufnahme von Elektronen infolge chemischer Wechselwirkung mit einem Reaktanden in einen energieärmeren Zustand unter Ausbildung stabiler Elektronenschalen überzugehen (siehe CD-Römpp Chemie Lexikon, "Oxidantien", Version 1, Stuttgart/New York, Georg Thieme Verlag 1995). Die Oxidationsmittel werden dabei reduziert.

Die beim erfindungsgemäßen Verfahren einsetzbaren Oxidationsmittel können etwa gasförmiger, flüssiger oder fester Natur sein. Sie können zum Beispiel in ladungsneutraler, ionischer oder zwitterionischer Form vorliegen. Bei den Oxidationsmitteln kann es sich ferner um Elemente oder um Verbindungen handeln. Bevorzugt sind Oxidationsmittel, welche nur eine sehr geringe Eigenfarbe besitzen oder farblos sind.

Als Beispiele ladungsneutraler Oxidationsmittel seien molekularer Sauerstoff (O₂), Ozon (O₃), Chlor (Cl₂), Halogenoxide (z.B. Chlordioxid), Hypohalogenige Säuren (z.B. Hypochlorige Säure), Halogenige Säuren (z.B. Chlorige Säure), Halogensäure (z.B. Chlorsäure), Perhalogensäuren (z.B. Perchlorsäure) und Peroxide, wie beispielsweise Wasserstoffperoxid, Hydroperoxide, "Peroxide" (R-O-O-R), Diacylperoxide, Persäuren, Persäureester, Ketonperoxide, Epidioxide, genannt. Als Beispiele ionischer Oxidationsmittel seien Peroxodisulfate (z.B. Natrium- oder Kaliumperoxodisulfat), Hypohalogenite (z.B. Natrium- oder Kaliumhypochlorit), Halogenite (z.B. Natrium- oder Kaliumchlorit), Halogenate (z.B. Natrium- oder Kaliumchlorat), Perhalogenate (z.B. Natrium- oder Kaliumperchlorat) oder Metallperoxide (z.B. Natriumperoxid oder Bariumperoxid) genannt.

Bevorzugt setzt man beim erfindungsgemäßen Verfahren ein sauerstoffübertragendes Oxidationsmittel und besonders bevorzugt molekularen Sauerstoff (O₂), Ozon (O₃), Peroxide oder Gemische davon ein. Als besonders bevorzugte Peroxide seien
- Wasserstoffperoxid,
- Hydroperoxide, wie z.B. tert.-Butylhydroperoxid oder Cumolhydroperoxid,
- "Peroxide" (R-O-O-R), wie z.B. Di-tert.-butylperoxid,
- Diacylperoxide, wie z.B. Dibenzoylperoxid,
- Persäuren, wie z.B. Perbenzoesäure oder Benzoylpercarbamidsäure, und
- Persäureester , wie z.B. Perbenzoesäure-tert.-butylester
genannt.

Ganz besonders bevorzugt ist der Einsatz von molekularem Sauerstoff (O₂), Wasserstoffperoxid und tert.-Butylhydroperoxid.

Die Oxidationsmittel können unverdünnt oder auch verdünnt mit weiteren Gasen, Flüssigkeiten (z.B. Lösungsmitteln) oder Feststoffen zugegeben werden. So kann beispielsweise molekularer Sauerstoff (O₂) als reines Sauerstoffgas oder verdünnt mit weiteren Gasen wie etwa Stickstoff, Edelgase (z.B. Argon, Helium, Neon), Kohlendioxid oder Wasserdampf zugegeben werden. Bei der Zugabe von molekularem Sauerstoff setzt man besonders bevorzugt Luft ein. Das für die Zugabe geeignete Ozon (O₃) kann beispielsweise durch Ozonisierung von molekularem Sauerstoff, insbesondere von reinem Sauerstoff oder von Luft, gewonnen werden.

Die Zugabe der Oxidationsmittel führt zu einer Stabilisierung und/oder Senkung der Farbzahl der Alkenylverbindung. Die genannte Farbzahl ist ein Kennwert für die Farbe transparenter Verbindungen. Je niedriger die Farbzahl, desto farbloser ist das Produkt. Weit verbreitet ist die Bestimmung der Farbzahl nach APHA, welche in DIN EN 1557 (März 1997) definiert ist.

Die beim erfindungsgemäßen Verfahren einzusetzenden Oxidationsmittel können beispielsweise chemisch oder physikalisch gelöst, emulgiert oder suspendiert in den Alkenylverbindungen vorliegen.

Die einzusetzende Menge des Oxidationsmittels ist in der Regel von der Natur und der Menge der farbgebenden Verunreinigungen, dem gewünschten Stabilisierungseffekt beziehungsweise der gewünschten Senkung der Farbzahl abhängig und durch einfache Experimente an das jeweilige System anzupassen. Im Allgemeinen stellt man einen Gehalt von 0,0001 bis 1000 Gew.-ppm und bevorzugt von 0,001 bis 500 Gew.-ppm an gelöstem Oxidationsmittel, bezogen auf die Alkenylverbindung ein.

Das erfindungsgemäße Verfahren wird im Allgemeinen bei einer Temperatur von 0 bis 100°C und bevorzugt von 10 bis 70°C durchgeführt, wobei die Alkenylverbindung bevorzugt in flüssiger Phase vorliegt. Es wird im Allgemeinen bei einem Druck von 0,01 bis 100 MPa abs, bevorzugt 0,05 bis 10 MPa abs und insbesondere bei Atmosphärendruck durchgeführt.

Beim Einsatz flüssiger oder fester Oxidationsmittel sowie deren Gemische oder Lösungen gibt man im Allgemeinen die gewünschte Menge zu der bevorzugt flüssigen Alkenylverbindung unter Vermischung hinzu. Beim Einsatz gasförmiger Oxidationsmittel führt man diese im Allgemeinen durch direktes Einleiten oder durch einfache Kontaktierung mit der bevorzugt flüssigen Alkenylverbindung zu. Die erwähnte Kontaktierung kann beispielsweise durch einfaches Überschichten der bevorzugt flüssigen Alkenylverbindung mit dem gasförmigen Oxidationsmittel oder durch gezieltes Einleiten des gasförmigen Oxidationsmittels in die bevorzugt flüssige Alkenylverbindung erfolgen.

Wird eine gezielte Senkung der Farbzahl gewünscht, so läßt man im Allgemeinen das Oxidationsmittel über einige Minuten bis wenige Tage einwirken. Die erforderliche Zeitdauer ist unter anderem abhängig von der Natur der farbgebenden Verunreinigungen, der Natur und der Konzentration des Oxidationsmittels und der gewünschten Farbzahl des behandelten Produkts. Werden gasförmige Oxidationsmittel, wie beispielsweise molekularer Sauerstoff (O₂) eingesetzt, so werden diese vorteilhafterweise über die Zeitdauer hinweg kontinuierlich oder periodisch zugegeben. Flüssige oder feste Oxidationsmittel werden in der Regel zu Beginn zugegeben, wobei natürlich auch weitere, spätere Zugaben möglich sind.

Wird in erster Linie eine Stabilisierung der Farbzahl gewünscht, so läßt man im Allgemeinen das Oxidationsmittel über einen längeren Zeitraum von Tagen, Wochen, Monaten oder Jahren einwirken. Die Alkenylverbindung kann somit auch über einen längeren Zeitraum hinweg gelagert oder transportiert werden. Wie allgemein bekannt ist, steigt die Farbzahl von Alkenylverbindungen ohne die erfindungsgemäße Maßnahme im Laufe der Zeit an. Unter einer Stabilisierung der Farbzahl im Sinne der vorliegenden Erfindung ist eine Farbzahlentwicklung der Alkenylverbindung zu verstehen, welche zu geringeren Farbzahlen führt als ohne die erfindungsgemäße Maßnahme. So kann bei einer Stabilisierung der Farbzahl die Farbzahl (i) langsamer ansteigen als ohne die erfindungsgemäße Maßnahme, (ii) nahezu unverändert bleiben oder (iii) im Laufe der Zeit absinken. Zur Stabilisierung der Farbzahl wirkt das Oxidationsmittel vorteilhafterweise über den gesamten Zeitraum ein. Werden gasförmige Oxidationsmittel, wie beispielsweise molekularer Sauerstoff (O₂) eingesetzt, so werden diese bevorzugt durch Hindurchleiten durch die bevorzugt flüssigen Alkenylverbindungen oder durch Überschichten mit einem Gaspolster in das Produkt eingebracht.

Die beim erfindungsgemäßen Verfahren eingesetzten Alkenylverbindungen können mit Stabilisatoren zur Unterbindung unerwünschter Reaktionen wie etwa Zersetzung, Oligomerisierung oder Polymerisierung versetzt sein. Als Beispiele geeigneter Stabilisatoren seien N,N'-Bis(1-methylpropyl)-1,4-phenylendiamin, welcher von der BASF AG unter dem Handelsnamen Kerobit® BPD vertrieben wird, Alkalihydroxide oder Phenothiazin-Derivate genannt. Sind die Alkenylverbindungen mit Stabilisatoren versetzt, so liegt deren Gehalt im Allgemeinen bei 1 bis 1000 Gew.-ppm, bevorzugt bei 1 bis 100 Gew.-ppm und besonders bevorzugt bei 5 bis 50 Gew.-ppm.

Die beim erfindungsgemäßen Verfahren einzusetzenden Alkenylverbindungen besitzen beispielsweise die allgemeinen Formeln (Ia) oder (Ib) 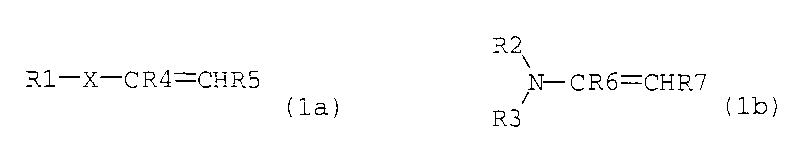 in denen X ein zweibindiges Heteroatom, R¹, R² und R³ jeweils unabhängig voneinander ein Kohlenstoff enthaltender organischer Rest, wobei R² und R³ auch miteinander verbunden sein können, und R⁴, R⁵, R⁶ und R⁷ jeweils unabhängig voneinander Wasserstoff oder ein Kohlenwasserstoffrest, bedeuten.

Unter einem Kohlenstoff enthaltenden organischen Rest ist ein unsubstituierter oder substituierter, aliphatischer, aromatischer oder araliphatischer Rest mit 1 bis 22 Kohlenstoffatomen zu verstehen. Dieser Rest kann ein oder mehrere Heteroatome, wie etwa Sauerstoff, Stickstoff oder Schwefel enthalten, beispielsweise - O-, -S-, -NR-, -CO- und/oder -N= in aliphatischen oder aromatischen Systemen, und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, Iod und/oder eine Cyanogruppe. Enthält der Kohlenstoff enthaltende organische Rest ein oder mehrere Heteroatome, so kann dieser auch über ein Heteroatom oder ein Heteroatom tragendes Kohlenstoffatom gebunden sein. Somit sind beispielsweise auch Reste, welche über ein Stickstoffatom oder eine CO-Gruppe gebunden sind, eingeschlossen.

Als bevorzugte einbindige, d.h. endständige Kohlenstoff enthaltende organische Reste für R¹, R² sowie R³ seien
* unverzweigtes oder verzweigtes, acyclisches und cyclisches Alkyl mit 1 bis 22 aliphatischen Kohlenstoffatomen, bei dem eine oder mehrere der -CH₂-Gruppen auch durch Heteroatome, wie -O-, oder durch Heteroatom enthaltende Gruppen, wie -COoder -NR-, ersetzt sein können und bei dem ein oder mehrere der Wasserstoffatome durch Substituenten, wie beispielsweise Arylgruppen ersetzt sein können,
* unverzweigtes oder verzweigtes, acyclisches und cyclisches Alkenyl mit 2 bis 22 aliphatischen Kohlenstoffatomen und einer oder mehrerer Doppelbindungen an beliebiger Stelle, bei dem eine oder mehrere der -CH₂-Gruppen auch durch Heteroatome, wie -O-, oder durch Heteroatom enthaltende Gruppen, wie -CO- oder -NR-, ersetzt sein können und bei dem ein oder mehrere der Wasserstoffatome durch Substituenten, wie beispielsweise Arylgruppen ersetzt sein können,
* Aryl mit bis zu 10 aromatischen Kohlenstoffatomen, bei dem eine oder mehrere der =CH-Gruppen durch Heteroatome, wie =N-, ersetzt sein können und bei dem eines oder mehrere der Wasserstoffatome durch Substituenten, wie beispielsweise Alkylgruppen ersetzt sein können,
* sowie Reste wie zuvor genannt, bei denen ein oder mehrere der Wasserstoffatome durch eine -X-CR4=CHR5 oder >Y-CR6=CHR7-Gruppe substituiert sind,
genannt.

Als bevorzugte zweibindige, d.h. verbundene Kohlenstoff enthaltende organische Reste für R²-R³ seien
* unverzweigtes oder verzweigtes Alkylen mit 3 bis 20 aliphatischen Kohlenstoffatomen, bei dem eine oder mehrere der - CH₂-Gruppen auch durch Heteroatome, wie -O-, oder durch Heteroatom enthaltende Gruppen, wie -CO- oder -NR-, ersetzt sein können und bei dem ein oder mehrere der Wasserstoffatome durch Substituenten, wie beispielsweise Arylgruppen ersetzt sein können,
* unverzweigtes oder verzweigtes Alkenylen mit 3 bis 20 Kohlenstoffatomen und einer oder mehrerer Doppelbindungen, bei dem eine oder mehrere der -CH₂-Gruppen auch durch Heteroatome, wie -O-, oder durch Heteroatom enthaltende Gruppen, wie -COoder -NR-, ersetzt sein können, bei dem des Weiteren eine oder mehrere der =CH-Gruppen durch Heteroatome, wie =N-, ersetzt sein können und bei dem ein oder mehrere der Wasserstoffatome durch Substituenten, wie beispielsweise Arylgruppen ersetzt sein können,
genannt.

Das bei den Alkenylverbindungen (Ia) genannte zweibindige Heteroatome X kann ein Sauerstoffatom oder ein Schwefelatom sein. Als Beispiele für die Alkenylverbindungen (Ia) seien Alkenylether, Alkenylester und Alkenylsulfide genannt. Bevorzugt werden beim erfindungsgemäßen Verfahren Alkenylverbindungen (Ia) eingesetzt, bei denen X Sauerstoff bedeutet.

Als Beispiele für die Alkenylverbindungen (Ib) seien Alkenylamine, N-Alkenylamide und N-Alkenylheterocyclen genannt. In der Bezeichnung N-Alkenylamide sind auch die cyclischen N-Alkenylamide, welche auch als N-Alkenyllactame bekannnt sind, umfasst.

Unter einem Kohlenwasserstoffrest ist ein aliphatischer, aromatischer oder araliphatischer Rest mit 1 bis 12 Kohlenstoffatomen zu verstehen. Als bevorzugte Kohlenwasserstoffreste für R⁴, R⁵, R⁶ und R⁷ seien C₁- bis C₄-Alkyl, wie beispielsweise Methyl, Ethyl, 1-Propyl, 2-Propyl, 1-Butyl, 2-Butyl, 2-Methyl-1-propyl und 2-Methyl-2-propyl, insbesondere Methyl, C₆-Aryl, konkret Phenyl, C₇- bis C₈-Aralkyl, wie beispielsweise Phenylmethyl und Phenylethyl, und C₇- bis C₈-Alkaryl, wie beispielsweise 2-Methylphenyl, 3-Methylphenyl und 4-Methylphenyl, genannt.

Besonders bevorzugt sind die Alkenylverbindungen (Ia) und (Ib), bei denen die Reste R⁴, R⁵, R⁶ und R⁷ unabhängig voneinander Wasserstoff oder Methyl bedeuten. Ganz besonders bevorzugt sind die Alkenylverbindungen (Ia) und (Ib), bei denen die Reste R⁴, R⁵, R⁶ und R⁷ Wasserstoff bedeuten, d.h. die Vinylverbindungen.

Als Beispiele der beim erfindungsgemäßen Verfahren einsetzbaren Alkenylsulfide der Formel (Ia) mit X gleich Schwefel seien Vinylmethyl-sulfid, Vinyl-ethyl-sulfid, Vinyl-(1-propyl)-sulfid, Vinyl-(2-propyl)-sulfid (Vinyl-isopropyl-sulfid), Vinyl-(1-butyl)-sulfid, Vinyl-(2-butyl)-sulfid (Vinyl-sek.-butyl-sulfid), Vinyl-(2-methyl-2-propyl)-sulfid (Vinyl-tert.-butyl-sulfid), Vinyl-pentyl-sulfid und dessen Isomere und Vinyl-hexyl-sulfid und dessen Isomere genannt.

Besonders bevorzugt handelt es sich beim erfindungsgemäßen Verfahren bei den Alkenylverbindungen (Ia) um Vinylether. Als Beispiele der bevorzugten Vinylether seien Vinyl-methyl-ether, Vinyl-ethyl-ether, Vinyl-(1-propyl)-ether, Vinyl-(2-propyl)-ether (Vinyl-isopropyl-ether), Vinyl-(1-butyl)-ether, Vinyl-(2-butyl)-ether (Vinyl-sek.-butyl-ether), Vinyl-(2-methyl-2-propyl)-ether (Vinyl-tert.-butyl-ether), Vinyl-pentyl-ether und dessen Isomere, Vinyl-hexyl-ether und dessen Isomere, Vinyl-heptylether und dessen Isomere, Vinyl-octyl-ether und dessen Isomere, Vinyl-nonyl-ether und dessen Isomere, Vinyl-decyl-ether und dessen Isomere, Vinyl-undecyl-ether und dessen Isomere, Vinyl-dodecyl-ether und dessen Isomere, Vinyl-tridecyl-ether und dessen Isomere, Vinyl-tetradecyl-ether und dessen Isomere, Vinyl-pentadecyl-ether und dessen Isomere, Vinyl-hexadecyl-ether und dessen Isomere, Vinyl-heptadecyl-ether und dessen Isomere, Vinyl-octadecyl-ether und dessen Isomere, Vinyl-nonadecyl-ether und dessen Isomere, Vinyl-eicosyl-ether und dessen Isomere, Vinyl-heneicosyl-ether und dessen Isomere, Vinyl-docosyl-ether und dessen Isomere, Vinyl-cyclopentyl-ether, Vinyl-cyclohexyl-ether, Vinyl-cycloheptyl-ether, Vinyl-cyclooctyl-ether, Vinyl-cyclododecylether, Vinyl-phenyl-ether, Vinyl-(2-methyl-phenyl)-ether, Vinyl-(3-methyl-phenyl)-ether, Vinyl-(4-methyl-phenyl)-ether, Vinyl-(phenyl-methyl)-ether, Vinyl-(2-phenyl-ethyl)-ether, 2-Hydroxyethyl-1-vinyl-ether (3-Oxa-penta-4-en-1-ol), Ethylenglykol-divinyl-ether (3,6-Dioxa-octa-1,7-dien), Diethylenglykol-monovinylether (3,6-Dioxa-octa-7-en-1-ol), Diethylenglykol-divinyl-ether (3,6,9-Trioxa-undeca-1,10-dien), Triethylenglykol-monovinyl-ether (3,6,9-Trioxa-undeca-10-en-1-ol), Triethylenglykol-divinyl-ether (3,6,9,12-Tetraoxa-tetradeca-1,13-dien), Tetraethylenglykol-monovinyl-ether (3,6,9,12-Tetraoxa-tetradeca-13-en-1-ol), Tetraethylenglykol-divinyl-ether (3,6,9,12,15-Pentaoxa-heptadeca-1,16-dien), 1,2-Propylenglykol-monovinyl-ether (4-Oxa-hexa-5-en-2-ol und 2-Methyl-3-oxa-penta-4-en-1-ol), 1,2-Propylenglykol-divinylether (4-Methyl-3,6-dioxa-octa-1,7-dien), 3-Hydroxypropyl-1-vinyl-ether (5-Oxa-hepta-6-en-1-ol), 1,3-Propylenglykol-divinylether (3,7-Dioxa-nona-1,8-dien), 4-Hydroxybutyl-1-vinyl-ether (5-Oxa-hepta-6-en-1-ol), 1,4-Butylenglykol-divinyl-ether (3,8-Dioxa-deca-1,9-dien), 5-Hydroxypentyl-1-vinyl-ether (6-Oxaocta-7-en-1-ol), 1,5-Pentylenglykol-divinyl-ether (3,9-Dioxa-undeca-1,10-dien), 6-Hydroxyhexyl-1-vinyl-ether (7-Oxa-nona-8-en-1-ol), 1,6-Hexylenglykol-divinyl-ether (3,10-Dioxa-dodeca-1,11-dien), 8-Hydroxyoctyl-1-vinyl-ether (9-Oxa-undeca-10-en-1-ol), 1,8-Octylenglykol-divinyl-ether (3,12-Dioxa-tetradeca-1,13-dien), 12-Hydroxydodecyl-1-vinyl-ether (13-Oxa-pentadeca-14-en-1-ol), 1,12-Dodecylenglykol-divinyl-ether (3,16-Dioxaoctadeca-1,17-dien), 4-Hydroxycyclohexyl-1-vinylether, 1,4-Cyclohexylen-divinyl-ether (1,4-Bis(vinyloxy)-cyclohexan), 4-Vinyloxyphenol und Bis(vinyloxy)-1,4-phenylen genannt.

Die beim erfindungsgemäßen Verfahren ganz besonders bevorzugten Vinylether sind Ethylenglykol-divinyl-ether (3,6-Dioxaocta-1,7-dien), Diethylenglykol-divinyl-ether (3,6,9-Trioxa-undeca-1,10-dien), Triethylenglykol-divinyl-ether (3,6,9,12-Tetraoxa-tetradeca-1,13-dien) und 4-Hydroxybutyl-1-vinyl-ether (5-Oxa-hepta-6-en-1-ol).

Besonders bevorzugt handelt es sich beim erfindungsgemäßen Verfahren bei den Alkenylverbindungen (Ib) um acyclische und cyclische N-Vinylamine, acyclische und cyclische N-Vinylamide und N-Vinylheterocyclen, insbesondere um N-Vinylamide und N-Vinylheterocyclen.

Als Beispiele der bevorzugten acylischen und cyclischen N-Vinylamine seien N-Vinyl-dimethyl-amin, N-Vinyl-diethyl-amin, N-Vinyldi-(1-propyl)-amin, N-Vinyl-di-(2-propyl)-amin (N-Vinyl-diisopropyl-amin), N-Vinyl-di-(1-butyl)-amin, N-Vinyl-di-(2-butyl)-amin (N-Vinyl-di-sek.-butyl-amin), N-Vinyl-di-(2-methyl-2-propyl)-amin (N-Vinyl-di-tert.-butyl-amin), N-Vinyl-methyl-ethyl-amin, N-Vinyl-methyl-(1-propyl)-amin, N-Vinyl-methyl-(2-propyl)-amin (N-Vinyl-methyl-isopropyl-amin), N-Vinyl-methyl-(1-butyl)-amin, N-Vinyl-methyl-(2-butyl)-amin (N-Vinyl-methyl-sek.-butyl-amin), N-Vinyl-methyl- (2-methyl-2-propyl)-amin (N-Vinyl-methyl-tert.-butylamin), N-Vinyl-methyl-pentyl-amin und dessen Isomere, N-Vinyl-methyl-hexyl-amin und dessen Isomere, N-Vinyl-methyl-heptyl-amin und dessen Isomere, N-Vinyl-methyl-octyl-amin und dessen Isomere, N-Vinyl-methyl-nonyl-amin und dessen Isomere, N-Vinyl-methyl-decyl-amin und dessen Isomere, N-Vinyl-methyl-undecyl-amin und dessen Isomere, N-Vinyl-methyl-dodecyl-amin und dessen Isomere, N-Vinyl-methyl-tridecyl-amin und dessen Isomere, N-Vinyl-methyltetradecyl-amin und dessen Isomere, N-Vinyl-methyl-pentadecylamin und dessen Isomere, N-Vinyl-methyl-hexadecyl-amin und dessen Isomere, N-Vinyl-methyl-heptadecyl-amin und dessen Isomere, N-Vinyl-methyl-octadecyl-amin und dessen Isomere, N-Vinyl-methyl-nonadecyl-amin und dessen Isomere, N-Vinyl-methyl-eicosyl-amin und dessen Isomere, N-Vinyl-methyl-heneicosyl-amin und dessen Isomere, N-Vinyl-methyl-docosyl-amin und dessen Isomere, N-Vinyl-methyl-cyclopentyl-amin, N-Vinyl-methyl-cyclohexyl-amin, N-Vinylmethyl-cycloheptyl-amin, N-Vinyl-methyl-cyclooctyl-amin, N-Vinylmethyl-cyclododecyl-amin, N-Vinyl-methyl-phenyl-amin, N-Vinyl-diphenyl-amin, N-Vinyl-methyl-(2-methyl-phenyl)-amin, N-Vinyl-methyl- (3-methyl-phenyl)-amin, N-Vinyl-methyl-(4-methyl-phenyl)-amin, N-Vinyl-methyl-(phenyl-methyl)-amin, N-Vinyl-methyl-(2-phenyl-ethyl)-amin, N-Vinyl-pyrrolidin, N-Vinyl-piperidin, N-Vinyl-morpholin genannt.

Als Beispiele der bevorzugten acyclischen und cyclischen N-Vinylamide seien N-Vinyl-N-methyl-acetamid, N-Vinyl-pyrrolidon, N-Vinyl-2-piperidon (N-Vinyl-δ-valerolactam), N-Vinyl-ε-caprolactam (N-Vinyl-6-aminohexansäure-lactam), N-Vinyl-7-aminoheptansäurelactam, N-Vinyl-8-aminooctansäure-lactam, N-Vinyl-9-aminononansäure-lactam, N-Vinyl-10-aminodecansäure-lactam, N-Vinyl-12-aminododecansäure-lactam (N-Vinyl-laurin-lactam) genannt.

Als Beispiele der bevorzugten N-Vinylheterocyclen seien N-Vinylpyrrol, N-Vinyl-pyrazol, N-Vinyl-imidazol, N-Vinyl-1,2,3-triazol, N-Vinyl-1,2,4-triazol, N-Vinyl-1,3,4-triazol und N-Vinyl-2-methylimidazol genannt, insbesondere N-Vinyl-imidazol.

Beim erfindungsgemäßen Verfahren ganz besonders bevorzugt ist der Einsatz von N-Vinyl-ε-caprolactam. Das N-Vinyl-ε-caprolactam kann dabei in fester Phase, in flüssiger Phase oder auch in einer Mischung beider Phasen vorliegen. Bevorzugt hält man das N-Vinyl-ε-caprolactam in flüssiger Phase, wobei besonders bevorzugt über 90 Gew.-%, ganz besonders bevorzugt über 99 Gew.-% und insbesondere das gesamte N-Vinyl-ε-caprolactam in der flüssigen Phase vorliegen.

Um das N-Vinyl-ε-caprolactam in flüssiger Phase zu halten, wendet man im Allgemeinen eine Temperatur an, die dem Schmelzpunkt entspricht oder oberhalb des Schmelzpunktes liegt. Reines N-Vinyl-ε-caprolactam besitzt einen Schmelzpunkt von 35°C. Bevorzugt hält man das N-Vinyl-ε-caprolactam beim erfindungsgemäßen Verfahren bei einer Temperatur von 35 bis 100°C, besonders bevorzugt bei 35 bis 75°C und ganz besonders bevorzugt bei 35 bis 60°C.

In einer Ausführungsform zur Senkung der Farbzahl leitet man über einen Zeitraum von mehreren Minuten bis wenigen Tagen Luft durch die flüssige Alkenylverbindung. Ist die gewünschte niedrigere Farbzahl erreicht, wird die Zufuhr von Luft unterbrochen und das Produkt kann beispielsweise weiterverarbeitet oder gelagert werden.

In einer Ausführungsform zur Stabilisierung der Farbzahl gibt man die Alkenylverbindung in einen Behälter und überschichtet das Produkt mit Luft. Die Alkenylverbindung kann nun im festen oder flüssigen Zustand gelagert beziehungsweise transportiert werden. In einer weiteren Ausführungsform zur Stabilisierung der Farbzahl löst man ein festes oder flüssiges Oxidationsmittel in der flüssigen Alkenylverbindung. Die Alkenylverbindung kann nun im festen oder flüssigen Zustand gelagert beziehungsweise transportiert werden.

In einer bevorzugten Ausführungsform zur Stabilisierung der Farbzahl von N-Vinyl-ε-caprolactam wird das flüssige Produkt in Behälter gefüllt, mit Luft überschichtet und bei 35 bis 60°C gelagert beziehungsweise transportiert.

In einer weiteren bevorzugten Ausführungsform zur Stabilisierung der Farbzahl von N-Vinyl-ε-caprolactam löst man ein festes oder flüssiges Oxidationsmittel (z.B. ein Peroxid) im flüssigen Produkt und lagert beziehungsweise transportiert das Produkt bei 35 bis 60°C im flüssigen Zustand.

Das erfindungsgemäße Verfahren zur Stabilisierung und/oder Senkung der Farbzahl von Alkenylverbindungen ist besonders überraschend, da diese polymerisationsempfindlich sind und nach allgemeinem Fachwissen gerade durch Oxidationsmittel unerwünschte und unkontrollierte Nebenreaktionen, wie beispielsweise Oligomerisierung oder Polymerisierung, zu erwarten sind. Nach dem allgemeinen Fachwissen wäre daher eine Erhöhung der Farbzahl zu erwarten. Der beobachtete gegenteilige Effekt einer Erniedrigung der Farbzahl ist vollkommen unerwartet.

Das erfindungsgemäße Verfahren ermöglicht die Stabilisierung und/oder Senkung der Farbzahl von Alkenylverbindungen unter Vermeidung eines hohen Aufwandes, wobei farbzahlstabilisierte Alkenylverbindungen mit sehr niedriger Farbzahl erhalten werden, welche auch nach längerer, mehrmonatiger Lagerung keine Tendenz zur Verfärbung zeigen.

### Beispiele

Zur Charakterisierung der möglichen Verfärbung der Alkenylverbindungen wurden die Farbzahlen nach APHA, analog DIN EN 1557 bestimmt.

### Beispiele 1 bis 2

Das in den Beispielen 1 bis 6 eingesetzte N-Vinyl-ε-caprolactam hatte eine Reinheit von 99,7 Gew.-% N-Vinyl-ε-caprolactam und war mit etwa 10 Gew.-ppm N,N'-Bis(1-methylpropyl)-1,4-phenylendiamin (Handelsname Kerobit® BPD) stabilisiert. Der Gehalt an restlichem ε-Caprolactam betrug etwa 0,3 Gew.-%.

Im Vergleichsbeispiel 1 wurde ca 900 g flüssiges N-Vinyl-ε-caprolactam mit einer Farbzahl nach APHA von 80 unter einer Stickstoff-Schutzgasatmosphäre in eine Stickstoff gefüllte 1000-ml Polyethylenflasche gegeben und verschlossen. Das N-Vinyl-ε-caprolactam wurde nun als unterkühlte Schmelze bei etwa 25°C mit dem darüberliegenden Stickstoffpolster gelagert. Nach neun Monaten wurde erneut die Farbzahl nach APHA bestimmt. Sie betrug 238.

Im erfindungsgemäßen Beispiel 2 wurden ca. 900 g des flüssigen N-Vinyl-ε-caprolactams mit einer Farbzahl nach APHA von 80 in eine luftgefüllte 1000-mL Polyethylenflasche gegeben und verschlossen. Das N-Vinyl-ε-caprolactam wurde nun als unterkühlte Schmelze bei etwa 25°C mit dem darüberliegenden Luftpolster gelagert. Jeden Monat wurde die Flasche für ca. 30 Sekunden geöffnet, um das Luftpolster zu erneuern. Nach neun Monaten wurde erneut die Farbzahl nach APHA bestimmt. Sie betrug 19.

Das Vergleichsbeispiel 1 zeigt, daß N-Vinyl-ε-caprolactam ohne Zusatz eines Oxidationsmittels eine stark ausgeprägte Tendenz zur Verfärbung besitzt und die Farbzahl nach APHA innerhalb von neun Monaten signifikant von 80 auf 238 anstieg. In Gegenwart eines Oxidationsmittels hingegen sank die Farbzahl deutlich von 80 auf 19, wie das erfindungsgemäße Beispiel 2 zeigt. Die Farbzahl beim erfindungsgemäßen Beispiel 2 betrug somit nur etwa 8% der Farbzahl im Vergleichsbeispiel 1. Beispiel 2 bestätigt sowohl eine deutliche Senkung der Farbzahl als auch eine ausgeprägte Farbzahlstabilisierung.

### Beispiel 3

Das N-Vinyl-ε-caprolactam aus dem Vergleichsbeispiel 1 mit einer Farbzahl nach APHA von 238 wurde auf 40°C aufgeheizt und über ein Einleitungsrohr über vier Tage hinweg Luft durchgeperlt. Die Farbzahl verringerte sich dabei auf 183, was einer Verringerung des Wertes um etwa ¼ entspricht.

### Beispiel 4

500 g N-Vinyl-ε-caprolactam (unstabilisiert, Farbzahl nach APHA von 45) wurden 95 Stunden bei 40 °C gelagert. Während dieser Zeit wurde ein Luftstrom durch das Produkt geleitet. Am Ende des Versuchs betrug die Farbzahl 22.

### Beispiel 5

500 g N-Vinyl-ε-caprolactam (stabilisiert mit 10 Gew.-ppm Kerobit, Farbzahl nach APHA von 45) wurden 71 Stunden bei 40 °C gelagert. Während der ersten 69 Stunden wurde ein Stickstoffstrom durch das Produkt geleitet. Die Farbzahl stieg daraufhin auf 75 an. Danach wurden noch 2 Stunden Luft hindurchgeleitet. Die Farbzahl fiel daraufhin auf 64.

### Beispiel 6

5 g N-Vinyl-ε-caprolactam (stabilisiert mit 10 Gew.-ppm Kerobit, Farbzahl nach APHA von 174) wurden mit 50 Gew.-ppm einer 30%igen wässrigen Wasserstoffperoxid-Lösung versetzt und bei 50°C 3 Tage aufbewahrt. Anschließend wurde eine Farbzahl nach APHA von 101 gemessen.

### Beispiel 7

Ein 220 L-Fass mit 190 kg Diethylenglycoldivinylether (Stickstoff überlagert, Farbzahl nach APHA von 667) wurde über das Spundloch (Öffnung ca. 10 cm) geöffnet und 11 Tage an Luft gelagert. Die Farbzahl nach APHA verringerte sich dabei auf 425.

## Patentansprüche

1. Verfahren zur Stabilisierung und/oder Senkung der Farbzahl von Alkenylverbindungen mit einem zwei- oder dreibindigen Heteroatom in α-Stellung zur Doppelbindung, **dadurch gekennzeichnet, daß** man die Alkenylverbindungen mit einem Oxidationsmittel versetzt.

2. Verfahren nach Anspruch 1, **dadurch gekennzeichnet, daß** man als Oxidationsmittel ein sauerstoffübertragendes Oxidationsmittel einsetzt.

3. Verfahren nach Anspruch 2, **dadurch gekennzeichnet, daß** man als sauerstoffübertragendes Oxidationsmittel molekularen Sauerstoff, Ozon, Wasserstoffperoxid, organische Peroxide oder Gemische davon einsetzt.

4. Verfahren nach den Ansprüchen 1 bis 3, **dadurch gekennzeichnet, daß** man einen Gehalt von 0,0001 bis 1000 Gew.-ppm an gelöstem Oxidationsmittels bezogen auf die Alkenylverbindung einstellt.

5. Verfahren nach den Ansprüchen 1 bis 4, **dadurch gekennzeichnet, daß** es sich bei den Alkenylverbindungen um Verbindungen der allgemeinen Formeln (Ia) oder (Ib) 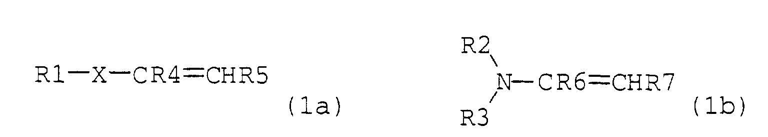 in denen X ein zweibindiges Heteroatom, R¹, R² und R³ jeweils unabhängig voneinander ein Kohlenstoff enthaltender organischer Rest, wobei R² und R³ auch miteinander verbunden sein können, und R⁴, R⁵, R⁶ und R⁷ jeweils unabhängig voneinander Wasserstoff oder ein Kohlenwasserstoffrest, bedeuten, handelt.

6. Verfahren nach den Ansprüchen 1 bis 5, **dadurch gekennzeichnet, daß** es sich bei den Alkenylverbindungen um Vinylverbindungen handelt.

7. Verfahren nach den Ansprüchen 5 bis 6, **dadurch gekennzeichnet, daß** es sich bei den Alkenylverbindungen (Ia) um Vinylether handelt.

8. Verfahren nach den Ansprüchen 5 bis 6, **dadurch gekennzeichnet, daß** es sich bei den Alkenylverbindungen (Ib) um N-Vinylamide oder N-Vinylheterocyclen handelt.

9. Verfahren nach Anspruch 8, **dadurch gekennzeichnet, daß** es sich bei den N-Vinylamiden um N-Vinyl-ε-caprolactam handelt.

10. Verfahren nach Anspruch 9, **dadurch gekennzeichnet, daß** man das N-Vinyl-ε-caprolactam bei einer Temperatur von 35 bis 60°C hält.
